# FASCICULE DE BREVET EUROPEEN

(11) **EP 3 615 993 B1**
(45) Date de publication et mention de la délivrance du brevet: **24.06.2026**
(21) Numéro de dépôt: 18723367.1
(22) Date de dépôt: 26.04.2018
(51) Int. Cl.: G02C 7/10, G02B 27/01

(54) **PROCEDE DE DETERMINATION D'UN FILTRE DE LUMIERE APPLIQUE A UN VERRE DE LUNETTES, DISPOSITIF D'AFFICHAGE ET CASQUE DE REALITE VIRTUELLE ASSOCIES**
VERFAHREN ZUR BESTIMMUNG EINES AUF EIN BRILLENGLAS ANGEWANDTEN LICHTFILTERS, ZUGEHÖRIGE ANZEIGEVORRICHTUNG UND VIRTUELLER REALITÄTSHELM
METHOD FOR DETERMINING A LIGHT FILTER APPLIED TO A SPECTACLE LENS, ASSOCIATED DISPLAY DEVICE AND VIRTUAL REALITY HELMET

(30) Priorité: 28.04.2017 FR 1753825
(43) Date de publication de la demande: 04.03.2020
(73) Titulaire: Essilor International, 94220 Charenton-le-Pont (FR)
(72) Inventeur: CHENE, Sylvain, 94220 Charenton-Le-Pont (FR); DEBIEUVRE, Amandine, 94220 Charenton-Le-Pont (FR); DUBAIL, Marie, 94220 Charenton-Le-Pont (FR); MARIE, Sarah, 94220 Charenton-Le-Pont (FR); MONTECELO, Susana, 94220 Charenton-Le-Pont (FR); SCHERLEN, Anne-Catherine, 94220 Charenton-Le-Pont (FR); ARROUY, Frédéric, 94220 Charenton-Le-Pont (FR)
(74) Mandataire: Jacobacci & Partners France
(86) Numéro de dépôt international: PCT/FR2018/051067
(87) Numéro de publication internationale: WO 2018/197820

(56) Documents cités:
- WO-A1-2014/174067
- WO-A1-2016/113506
- WO-A1-99/34246
- US-A- 5 714 967
- US-A- 6 099 126
- US-A1- 2012 274 902
- US-A1- 2015 049 304
- US-A1- 2016 157 712
- ANONYMOUS: "Luminance", LIGHTING DESIGN GLOSSARY, 23 October 2013 (2013-10-23), XP055885545, Retrieved from the Internet <URL:https://web.archive.org/web/20131023183606/https://www.schorsch.com/en/kbase/glossary/luminance.html> [retrieved on 20220131]

## Description

### DOMAINE TECHNIQUE AUQUEL SE RAPPORTE L'INVENTION

La présente invention concerne de manière générale le domaine de l'optique ophtalmique.

Elle concerne en particulier le domaine de la prescription d'un filtre de lumière à un individu porteur de lunettes avec ledit filtre.

Elle concerne plus particulièrement un procédé de détermination d'un filtre de lumière adapté à être appliqué à un verre de lunettes pour améliorer ou maintenir le confort visuel et/ou les performances visuelles de cet individu.

Elle concerne également un dispositif d'affichage et un casque de réalité virtuelle adaptés à la mise en œuvre de ce procédé.

### ARRIERE-PLAN TECHNOLOGIQUE

Généralement, un individu devant déterminer quel est le meilleur filtre pour ses besoins et usages effectue son choix sur la base de critères principalement esthétiques (couleur ou teinte du verre, design de la monture, ...) ou financiers (prix du verre recevant le filtre et/ou de la monture destinée à accueillir le verre).

Pour une prescription de verres solaires par exemple, l'un des rares critères techniques également considéré par l'individu concerne la « classe » du verre telle que définie par la norme NF EN ISO 12312-1. Cette classe peut aller : de 0 pour un verre *« clair »* transmettant entre 80 et 100 % de la lumière visible entre 380 nanomètres (nm) et 780 nm et 8 à 10 % de la lumière ultra-violette (UV) dans la gamme « UVB » entre 280 nm et 315 nm) ; à 4 pour un verre extrêmement sombre ne transmettant qu'entre 3 et 8% de la lumière visible et moins de 0,3 à 0,8 % de la lumière UVB.

Toutefois, les critères esthétiques et financiers ou bien la classe du filtre ne sont pas réellement pertinents pour permettre à l'individu de déterminer le filtre de lumière le plus adapté pour améliorer ou maintenir son confort visuel et/ou ses performances visuelles.

En effet, chaque individu, qu'il soit jeune ou âgé, selon ses habitudes de vie (en intérieur ou en extérieur, travail sur écran ou travail minutieux, etc...), en fonction des conditions lumineuses les plus fréquentes auxquelles il est confronté (environnement très lumineux, environnement de nuit, etc...), en fonction de ses besoins particuliers (besoin de protection contre l'éblouissement ponctuel, besoin de protection de son acuité visuelle), etc... éprouve une sensibilité qui lui est propre.

Par ailleurs, la prescription de filtre de lumière pour un individu, qu'il s'agisse entre autres de filtres solaires (par exemple des verres solaires teintés) ou bien de filtres sous la forme d'un revêtement spécifique sur verre de lunettes (par exemple un revêtement anti-reflet ou bien un traitement anti-UV), est une tâche techniquement difficile pour les professionnels de la vision (opticiens, optométristes, etc...).

En effet, celle-ci nécessite généralement la prise de nombreuses mesures relatives à la sensibilité propre du porteur auquel ledit filtre se destine.

On connaît aussi du document US 2015/049304 un procédé de fabrication d'un verre de lunettes ophtalmiques correctives personnalisé pour un porteur.

On connaît notamment du document de brevet FR 1650383 au nom de la demanderesse un procédé de détermination d'un filtre pour une lentille ophtalmique destinée à être placée devant l'œil d'un porteur, ledit filtre étant apte à améliorer ou à maintenir le confort visuel et/ou les performances visuelles dudit porteur.

Le procédé de détermination du document FR 1650383 comporte une étape de mesure d'une grandeur représentative d'une sensibilité de l'œil du porteur à un flux lumineux caractéristique, et une étape de détermination d'au moins une caractéristique optique dudit filtre en fonction de la grandeur représentative mesurée.

Par *« flux lumineux caractéristique »,* le document FR 1650383 entend :
- soit un flux lumineux *« réel »* auquel est soumis le porteur dans une tâche donnée : le flux lumineux caractéristique est alors caractéristique de l'environnement lumineux ambiant dans lequel le porteur se retrouvera pour la réalisation de la tâche visuelle ;
- soit à un flux lumineux *« artificiel »* en ce sens qu'il reproduit au moins partiellement le flux lumineux auquel sera soumis le porteur : le flux lumineux caractéristique est alors représentatif d'au moins une source lumineuse d'inconfort visuel ou de perte de performances visuelles pour le porteur.

Si le procédé du document FR 1650383 permet de déterminer précisément et objectivement la ou les caractéristiques optiques d'un ou plusieurs filtres tout à fait adaptés au porteur, il nécessite cependant une mise en œuvre fastidieuse, en particulier parce qu'il est nécessaire de générer un flux lumineux caractéristique apte à tester la sensibilité de l'œil du porteur à la lumière.

En effet, comme l'enseigne le document FR 1650383, la complexité de la détermination du filtre réside dans le fait que cette sensibilité à la lumière de l'œil du porteur est dépendante à la fois des caractéristiques physiques ou optiques du flux lumineux caractéristique, de la physiologie du système visuel du porteur, et de l'impact fonctionnel d'un flux lumineux gênant sur les performances visuelles ou le confort visuel du porteur dans une tâche visuelle donnée.

De plus, l'interprétation des mesures de sensibilité, en particulier les données physiologiques, pour déterminer quel est le bon filtre de lumière requiert une grande expérience de la part de l'opticien.

### OBJET DE L'INVENTION

Afin de remédier à l'inconvénient précité de l'état de la technique, la présente invention propose un procédé permettant de déterminer quel est le filtre de lumière optimal pour un individu sans requérir la prise de mesures spécifiques complexes relatives à la sensibilité dudit individu à la lumière ou à un type de lumière.

Un autre objectif de l'invention est d'aider un professionnel de la vision à conseiller un filtre de lumière adapté et à argumenter à propos de son bénéfice et de le faire tester rapidement.

Plus particulièrement, on propose selon l'invention un procédé de détermination d'un filtre de lumière adapté à être appliqué à un verre de lunettes pour améliorer ou maintenir le confort visuel et/ou les performances visuelles d'un individu porteur desdites lunettes comme décrit dans l'objet de la revendication 1.

Ainsi, grâce à l'invention, il est possible de déterminer un filtre de lumière de manière précise dans des conditions réalistes pour l'individu.

L'invention présente l'avantage de mettre en situation le futur porteur du filtre dans un environnement lumineux prédéterminé avec un scénario donné choisis en fonction de ses besoins ou usages, et de lui proposer un rendu réaliste du filtre d'essai, c'est-à-dire de l'immerger réellement (cas d'un filtre d'essai *réel*) ou virtuellement (cas d'un filtre d'essai *virtuel* simulé) dans des conditions visuelles identiques ou *quasi* identiques à celles qu'il expérimenterait dans la réalité avec ce filtre d'essai.

L'invention définit un ensemble d'étapes de mise en situation, qui permet d'évaluer le rendu et/ou le bénéfice apporté par le filtre d'essai sélectionné selon un ou plusieurs critères prédéterminés, d'aider à la prescription du ou des filtres de lumières optimaux, et de les différencier.

L'évaluation sur la base du ou des critères prédéfinis en fonction des besoins ou usages permet une prescription adaptée du bon filtre de lumière à l'individu.

Elle offre un nouveau service pour le professionnel de la vision qui peut facilement proposer et personnaliser des filtres de lumière selon la sensibilité visuelle de son client.

Par données relatives aux « usages » de l'individu, on entend toutes les données concernant les utilisations que l'individu pourra faire de son filtre de lumière. Ces données concernent par exemple les conditions d'utilisation du filtre de lumière :
- utilisation en intérieur ou en extérieur ;
- utilisation de jour ou de nuit ;
- utilisation ponctuelle ou prolongée, fréquence et durée d'utilisation ;
- utilisation pour la conduite d'un véhicule ou d'une machine ;
- utilisation dans un lieu spécifique : mer ou montagne (lumière plus ou moins polarisée), lieu généralement fortement ensoleillé ou bien souvent couvert, lieu où la température ambiante est élevée/faible, ... ;
- utilisation en combinaison avec un autre dispositif visuel particulier : lunettes de sécurité, loupes, ... ;
- utilisation pour une activité ou une tâche spécifique : sport, télévision, lecture/écriture, conduite automobile, travail sur écran, travail manuel, ....

Par données relatives aux *« besoins »* de l'individu, on entend toutes les données concernant les déficiences ou gênes visuelles de l'individu, et éventuellement les symptômes qui y sont associés, que le filtre de lumière recherché est sensé pallier, telles que celles citées non-limitativement ci-après :
- inconfort visuel ;
- sensibilité à l'éblouissement pour un certain type de source lumineuse : source plus ou moins intense, ponctuelle ou étendue, directe ou indirecte, polarisée ou non, permanente ou transitoire, diffuse ou angulairement sélective ;
- sensibilité à la lumière dans des conditions particulières : de jour ou de nuit, avec ou sans correction visuelle, ... ;
- gêne par une source de lumière émettant dans une gamme de longueur d'onde donnée : ultra-violet, visible, lumière bleue entre 400 nm et 460 nm, ... ;
- perte dans la perception : des contrastes, des mouvements, de la profondeur, ... ;
- problème dans la vision mono- ou binoculaire, telle qu'une pathologie de type dégénérescence maculaire liée à l'âge (DMLA) ou glaucome ;
- diminution du champ visuel attentionnel central et/ou périphérique ;
- difficulté dans la reconnaissance des formes ou des objets ;
- dégradation de l'acuité visuelle, amétropie ;
- rétrécissement du champ visuel ;
- diminution dans la performance de lecture ;
- fatigue visuelle ;
- temps de réaction du système visuel, temps de latence ou temps de récupération de la pupille, réponse pupillaire ;
- mauvaise perception des couleurs ; ou
- migraines, crise d'épilepsie.

Selon certains modes de réalisation du procédé de l'invention, l'image (ou le film) et la source lumineuse peuvent être sur un même support, i.e. générées par le même dispositif, par exemple un écran HDR (pour *« High Dynamic* Range *»* en anglais).

Selon d'autres modes de réalisation, l'image et la source peuvent être sur des supports différents, c'est-à-dire générées par deux dispositifs lumineux distincts, par exemple par un écran LCD (*« Liquid Crystals Display »*) classique et par une dalle lumineuse (*« Backlight Unit »*) respectivement.

De préférence, à l'étape a), on soumet ledit individu à un questionnaire comportant des questions relatives aux besoins et/ou aux usages de l'individu (voir ci-dessus).

Les réponses aux questions de ce questionnaire constituent alors les données collectées de l'étape a).

Avantageusement, on peut également recueillir, à l'étape a), des mesures relatives à l'environnement lumineux de l'individu au moyen d'au moins un capteur de lumière.

De façon non limitative, on entend par mesures relatives à l'environnement lumineux de l'individu, toute mesure physique, en particulier optique, de la luminance visuelle perçue par l'individu, de l'éclairement rétinien, du spectre de la lumière ambiante, de la distribution angulaire et/ou spatiale des sources lumineuses de l'environnement, ....

Ledit capteur de lumière comprend tout capteur sensible à la puissance (lumineuse), à la luminance, à l'éclairement, au spectre ou à la température de couleur de ces sources lumineuses.

Ensuite, à l'étape b1), ledit filtre d'essai est sélectionné sur la base d'un critère d'évaluation.

Avantageusement, ledit filtre d'essai est sélectionné à l'étape b1) en fonction desdites données collectées à l'étape a). Ceci permet de faire une présélection pertinente du filtre d'essai.

Dans un exemple ne faisant pas partie de l'invention, ledit critère prédéterminé est choisi parmi l'un des critères suivants d'évaluation de filtre ou selon une combinaison de plusieurs de ces critères : le confort visuel, la sensibilité à l'éblouissement, la perception des couleurs, la perception des contrastes, la perception des mouvements, la perception de la profondeur, le champ visuel, la performance de lecture, la fatigue visuelle, le temps de réaction, la détection et/ou la reconnaissance de formes, la réponse pupillaire, le champ visuel attentionnel central et/ou périphérique.

Un exemple de combinaison de deux critères est l'acuité visuelle mesurée à bas contraste.

Aussi dans un exemple ne faisant pas partie de l'invention, le filtre d'essai peut également être choisi à l'étape b1) en fonction d'une caractéristique optique particulière, par exemple en fonction de l'un des paramètres suivants :
- un niveau de transmission lumineuse ou visuelle pour au moins une longueur d'onde ou un état de polarisation de la lumière, pour une bande de longueur d'onde, ou pour tout le domaine visible par exemple ;
- une réponse spectrale ou un spectre de réflexion et/ou de transmission sur une bande de longueur d'onde donnée, par exemple dans le domaine UV (UVA et/ou UVB) ou visible ;
- une variation spatiale des paramètres ci-dessus sur la surface de ladite lentille destinée à accueillir le filtre de lumière ;
- une variation temporelle de ces paramètres au cours du temps ; ou
- une variation de la teinte ou du niveau d'absorption en fonction d'un flux de lumière UV ou d'une variable électrique (courant, tension).

Avant ou après l'étape b1), il est prévu une deuxième étape de sélection b2) lors de laquelle on génère au moins une scène lumineuse comprenant :
- une image ou un film déterminé(e) en fonction des besoins et/ou des usages visuels de l'individu ; et
- au moins une source lumineuse génératrice d'un inconfort et/ou d'une perte de performances visuelles pour l'individu.

De préférence, l'image ou le film déterminé(e) en fonction des besoins et/ou des usages visuels récoltés lors de l'étape a) correspond à une image ou un film qui est représentatif(ve) :
- de l'utilisation projetée du filtre de lumière ;
- des environnements lumineux dans lesquels le porteur est susceptible d'évoluer lors du port de son filtre de lumière ; et/ou
- des gênes rencontrées et rapportées par l'individu.

Par exemple, si on collecte à l'étape a) du procédé une donnée indiquant qu'il portera son ou ses filtres lorsqu'il travaille de nuit, l'image ou le film représentera une situation de nuit ou une situation dans laquelle le niveau et/ou le spectre de luminance visuelle est faible et/ou décalé dans le bleu.

Encore à titre d'exemple, si l'individu rapporte une utilisation principale de son filtre lorsqu'il conduit son véhicule automobile, on prévoira alors une image ou un film représentant une situation de conduite (avec par exemple des panneaux ou feux de signalisation de différentes couleurs/formes/tailles/lisibilité, d'autres véhicules venant en sens inverse avec des feux de croisement allumés, etc...).

Toujours à titre d'exemple, si le porteur se plaint d'une gêne liée à sa trop grande sensibilité à l'éblouissement (avec par exemple des problèmes de temps de récupération rétinien élevé ou de perte d'acuité visuelle) quelle que soit la situation ou son activité, on pourra alors sélectionner une scène lumineuse dans laquelle l'image représentera un test visuel particulier permettant d'estimer le temps de récupération rétinien ou l'acuité visuelle.

De la même manière, la ou les sources lumineuses qui est/sont génératrice(s) chacune d'un inconfort et/ou d'une perte de performances visuelles pour l'individu est/sont représentative(s) des sources de gêne visuelle rapportées à l'étape a).

Par exemple, si on collecte à l'étape a) une donnée indiquant que l'individu est essentiellement gêné de nuit par les sources directes de lumière, la source lumineuse additionnelle dans la scène lumineuse (comprenant une image ou un film représentatif/ve d'une situation de nuit) sera caractéristique d'une source directe de lumière (par exemple les phares d'une voiture, la lumière d'un lampadaire urbain, ...).

À l'issue des étapes de sélection b1) et b2) du procédé de l'invention, il est alors prévu lors de l'étape c) de visualiser le rendu ou le bénéfice du ou des filtres d'essai sélectionnés selon différents critères prédéfinis d'évaluation.

En d'autres termes, on présente visuellement à l'individu (i.e. on met sous ou devant les yeux de l'individu) ladite scène lumineuse sélectionnée à l'étape b2) au travers dudit filtre d'essai sélectionné à l'étape b1).

De préférence, à l'étape c), ladite scène lumineuse sélectionnée à l'étape b2) est affichée par un dispositif d'affichage, et ledit individu porte réellement ledit filtre d'essai ou bien porte virtuellement ledit filtre d'essai, ladite scène lumineuse étant alors affichée par ledit dispositif d'affichage telle qu'elle serait vue par ledit individu s'il portait réellement ledit filtre d'essai, et ledit individu observe ledit dispositif d'affichage.

En d'autres termes, dans un mode de réalisation particulier, à l'étape c), ladite scène lumineuse sélectionnée à l'étape b2) est affichée par un dispositif d'affichage ; et ledit individu porte physiquement ledit filtre d'essai pour observer ledit dispositif d'affichage.

Dans un autre mode de réalisation préférée, à l'étape c), ladite scène lumineuse visionnée au travers dudit filtre d'essai sélectionné à l'étape b1) est simulée et affichée par un dispositif d'affichage telle que ladite scène lumineuse serait vue par ledit individu s'il portait virtuellement ledit filtre d'essai ; et ledit individu observe ledit dispositif d'affichage. Dans ce cas, l'effet du filtre d'essai est visualisé indirectement *via* l'affichage de la scène visuelle simulée et affichée.

Le but de l'étape d) est que l'individu porteur du filtre d'essai (réellement ou virtuellement) d'évaluer le bénéfice du filtre d'essai selon les critères sélectionnés à l'étape b1). Il s'agira d'évaluer l'acuité visuelle du porteur en situation. Le sujet peut ou pas être équipé de sa réfraction.

L'objectif est de définir des critères d'évaluations du filtre qui sont soit basés sur des tests génériques, soit basés sur le type d'environnement ou des besoins identifiés par le porteur.

Les critères d'évaluations peuvent être objectifs, subjectives ou mixtes. Pour le critère selon l'invention, on compare le résultat de l'évaluation de l'étape d) avec un seuil prédéterminé d'acuité visuelle.

En fonction des besoins et usages, ou encore en fonction du critère prédéfini d'évaluation, à l'étape e), le seuil prédéterminé est un seuil absolu ou bien un seuil relatif déterminé à partir d'un filtre de référence ou à partir du filtre d'essai précédemment testé, ou bien à partir d'une situation dans laquelle l'individu ne porte aucun filtre.

Le filtre de référence peut être un filtre médian en ce sens qu'il satisfait le besoin visuel de la moitié d'une population représentative d'individus.

Le filtre de référence peut encore être :
- le filtre que porte usuellement le porteur (filtre actuel) ;
- le filtre offrant la meilleure performance pour le critère choisi ; ou
- le dernier filtre lancé sur le marché.

L'étape finale de détermination du filtre de lumière permet de valider ou non la prescription du filtre.

Selon un mode de réalisation particulier de l'invention, on retient, à l'étape f), le filtre d'essai comme étant ledit filtre de lumière adapté si la comparaison de l'étape e) montre une amélioration dudit critère prédéfini à l'étape b1).

Selon un autre mode de réalisation particulier de l'invention, si la comparaison de l'étape e) montre, à l'inverse, une dégradation du critère prédéfini à l'étape b1), on reprend, après l'étape f), le procédé de détermination à l'étape b1) en sélectionnant un autre filtre d'essai, selon le même critère prédéfini ou bien selon un autre critère prédéfini d'évaluation. Dans ce cas, on peut prévoir à l'étape b2) de sélectionner une scène lumineuse identique ou bien différente, par exemple si un nouveau critère prédéfini d'évaluation a été sélectionné.

Ainsi, le procédé de détermination peut être un procédé itératif tendant vers le filtre de lumière optimal pour l'individu en fonction de ses besoins et/ou usages.

Afin de mettre en œuvre le procédé de détermination décrit ci-dessus, l'invention concerne également un dispositif d'affichage comme décrit dans l'objet de la revendication 10. Le dispositif d'affichage est destiné à tester le confort visuel et/ou les performances visuelles d'un individu comportant un écran graphique commandé par un micro-processeur pour afficher, sur ledit écran graphique, une image ou un film susceptible d'être visionnée par ledit individu testé.

Selon l'invention, ledit dispositif d'affichage comporte en outre un système d'illumination additionnel commandé par un autre micro-processeur ou par le même micro-processeur pour diffuser, audit individu observant ledit écran graphique, un flux lumineux additionnel présentant une énergie, une répartition spectrale, une variation temporelle, une distribution spatiale, et/ou une distribution angulaire prédéterminée(s), ledit système d'illumination additionnel étant agencé par rapport audit écran graphique de manière à laisser visible tout ou partie de ladite image ou dudit film.

Le dispositif d'affichage est particulièrement adapté à mettre en œuvre certains mode de réalisation du procédé de détermination précité.

De manière préférée, ledit dispositif d'affichage observé par l'individu comprend un système à réalité augmentée, un système à réalité virtuelle, un système de projection d'images, ou bien un écran graphique combiné avec un système d'illumination additionnel.

Selon l'invention, ledit système d'illumination additionnel du dispositif d'affichage selon l'invention est adapté à diffuser un flux lumineux additionnel permettant, en combinaison avec ledit écran graphique, d'atteindre une luminance visuelle adaptée à créer un inconfort visuel pour l'individu, en particulier un inconfort par éblouissement.

De préférence, la luminance visuelle atteinte, grâce à l'addition de la scène lumineuse diffusée par l'écran graphique et au flux lumineux additionnel diffusé par le système d'illumination additionnel, est supérieure ou égale à 1000 candelas par mètre-carré (cd/m²), mieux supérieure ou égale à l'une des valeurs suivantes : 1500, 2000, 3000 cd/m², encore mieux supérieure ou égale à 4000 cd/m².

Dans un mode de réalisation, le système lumineux additionnel conduit à lui seul à une luminance visuelle dans la gamme de 1000 cd/m² à 20000 cd/m², de préférence de 2000 cd/m² à 20000 cd/m², et mieux de 3000 cd/m² à 20000 cd/m².

De préférence encore, la luminance visuelle atteinte grâce au système d'illumination additionnel est celle obtenue grâce à une source lumineuse étendue et substantiellement plane qui présente une surface lumineuse pouvant atteindre un éclairement supérieure ou égale à 10000 lux.

De manière générale, il est difficile de trouver des écrans graphiques de prix et d'encombrement raisonnables qui affichent une luminance visuelle supérieure à 900 cd/m², la luminance visuelle standard étant de l'ordre de 300 à 500 cd/m².

Ainsi, grâce au dispositif d'affichage de l'invention, il est possible d'associer un écran graphique destiné à afficher l'image de la scène visuelle du procédé et un système d'illumination additionnel permettant d'atteindre, en combinaison avec l'écran graphique ou de préférence seul, des niveaux de luminance visuelle très importants, en tout cas suffisamment importants pour générer des conditions d'éblouissement chez un individu standard.

Il s'agit de permettre de simuler à un individu grâce au dispositif d'affichage un environnement lumineux de manière la plus réaliste possible.

Les dispositifs d'affichage actuels ne permettent pas de simuler correctement les flux lumineux très élevés, du fait de la luminosité limitée de leur écran graphique.

Il n'est donc pas possible de montrer à l'individu de manière réaliste les effets d'un filtre, ni de déterminer précisément sa sensibilité à un niveau de lumière élevé.

Le système d'illumination additionnel du dispositif d'affichage peut comporter au moins une source lumineuse comprenant des diodes électro-luminescentes (LED), des fibres optiques, ou des diodes électro-luminescentes organiques (OLED).

On peut également prévoir que le dispositif d'affichage comprenne des moyens de fixation du système d'illumination additionnel à l'écran graphique, par exemple des moyens de clipsage.

De préférence, le système d'illumination additionnel laisse apparaitre tout ou partie des informations (image, film, ...) affichées sur l'écran graphique du dispositif d'affichage.

Selon des examples ne faisant pas partie de l'invention, ledit système d'illumination additionnel comprend des diodes électroluminescentes et un film diffusant actif ou passif s'interposant entre les yeux dudit individu et ledit écran graphique, ledit film diffusant étant adapté à rétrodiffuser le flux lumineux émis par lesdites diodes électroluminescentes ;
- ledit système d'illumination additionnel comprend une dalle lumineuse placée devant ledit écran graphique, et un diffuseur, actif ou passif, en transmission s'interposant entre ladite dalle lumineuse et les yeux dudit individu ;
- ledit système d'illumination comprend au moins une diode électroluminescente et un miroir semi-réfléchissant s'interposant entre les yeux dudit individu et ledit écran graphique pour réfléchir le flux lumineux émis par ladite diode électroluminescente vers les yeux de l'individu.

D'autres caractéristiques non limitatives et avantageuses du dispositif d'affichage conforme à l'invention sont les suivantes:
- ledit dispositif d'affichage comporte au moins deux capteurs de lumière adaptés à délivrer un signal représentatif du niveau d'éclairement au niveau des deux yeux dudit individu, et dans lequel le niveau moyen de luminance de ladite image ou dudit film et/ou dudit flux lumineux additionnel est commandé en fonction de ce signal représentatif.

L'invention propose enfin un casque de réalité virtuelle destiné à être porté par un individu comportant :
- un dispositif d'affichage tel que défini ci-dessus ;
- des moyens pour maintenir ce dispositif d'affichage devant les yeux dudit individu ; et
- des moyens pour isoler ledit individu de la lumière ambiante.

De manière préférée, ledit micro-processeur du casque est adapté pour afficher une image (ou un film) formée d'une image (film) gauche pour l'œil gauche et d'une image (film) droite pour l'œil droit dudit individu, et le casque comporte également des moyens optiques de visualisation tridimensionnelle adaptés pour présenter ladite image gauche, respectivement ladite image droite, à l'œil gauche de l'individu, respectivement à l'œil droit de l'individu, de sorte que ledit individu visualise une image ou un film tridimensionnelle par fusion desdites images (films) gauche et droite.

Ces moyens optiques de visualisation tridimensionnelle peuvent par exemple comprendre deux lentilles minces dont les axes optiques sont parallèles et séparés d'une distance fixe ou variable, par exemple égale à l'écart pupillaire de l'individu.

Ces moyens optiques de visualisation tridimensionnelle peuvent comprendre deux groupes d'au moins deux lentilles permettant d'ajuster la puissance optique dudit groupe, par exemple pour s'adapter à la réfraction de l'individu, avec ou sans lunettes.

Ces moyens optiques de visualisation tridimensionnelle pourraient également comprendre simplement deux ouvertures circulaires adaptées pour chacun des deux yeux de l'individu et une cloison sensiblement plane placée entre les deux ouvertures, et s'étendant perpendiculairement au segment joignant deux points particuliers des yeux de l'individu, par exemple le centre de rotation de l'œil droit et le centre de rotation de l'œil gauche.

### DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :
- la figure 1 est un diagramme schématique représentant les différentes étapes du procédé de détermination selon l'invention ;
- la figure 2 est une vue schématique d'un casque de réalité virtuelle comportant un smartphone et un système d'illumination additionnel selon un premier mode de réalisation ;
- les figures 3 et 4 sont des vues de détail d'un diffuseur du système d'illumination additionnel de la figure 2 ;
- la figure 5 est une vue schématique d'un système d'illumination additionnel selon un deuxième mode de réalisation ;
- la figure 6 est une vue schématique d'un système d'illumination additionnel selon un troisième exemple ne pas couvert par l'invention ; et
- la figure 7 est une vue schématique d'un système d'illumination additionnel selon un quatrième exemple ne pas couvert par l'invention.

En préambule, on notera que les éléments identiques ou similaires des différents modes de réalisation représentés sur les différentes figures seront référencés par les mêmes signes de référence et ne seront pas décrits à chaque fois.

### Procédé

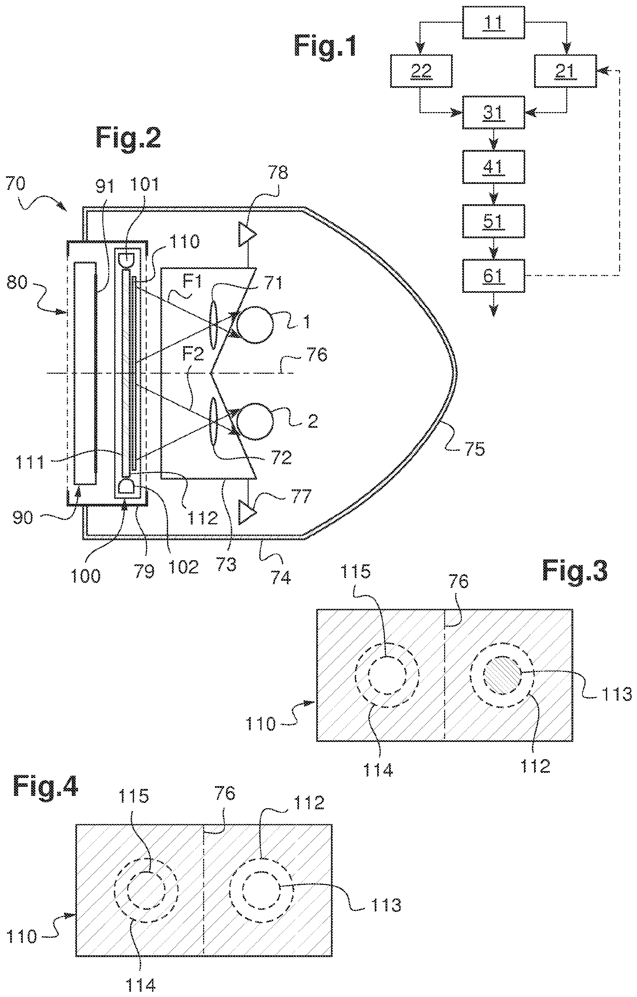

Sur la figure 1, on a représenté un diagramme schématique représentant les différentes étapes d'un procédé de détermination selon l'invention, étapes qu'on va détailler ci-après.

Ce procédé vise à trouver un filtre de lumière optimal pour un individu, ce filtre ayant pour fonction d'améliorer ou de maintenir le confort visuel et/ou les performances visuelles de cet individu lorsque celui-ci porte une paire de lunettes, sur l'un des verres de lunettes duquel, de préférence sur les deux, est appliqué ledit filtre optimal.

### Étape a (bloc 11 de la figure 1)

L'étape a), est une étape de collecte de données relatives aux besoins et usages de l'individu.

Comme expliqué précédemment, les données relatives aux « usages » de l'individu comprennent des informations sur les conditions d'utilisation du filtre de lumière envisagées par l'individu et sur les usages possibles qui ont été cités précédemment.

Par données relatives aux *« besoins »* de l'individu, on entend notamment toutes les données concernant les déficiences ou gênes visuelles de l'individu (et éventuellement les symptômes qui y sont associés) que le filtre de lumière recherché est sensé pallier, telles que celles déjà citées précédemment, de manière non limitative.

Les données relatives aux besoins peuvent également comprendre des informations sur le profil personnel de l'individu : son âge, son sexe, sa couleur des yeux, ses éventuelles pathologies visuelles actuelles ou passées (par ex. cataracte, densité de pigment maculaire, diffusion intraoculaire, DMLA, amblyopie, nystagmus, etc...), ses antécédents médicaux (par ex. : dyslexie, épilepsie, migraines, troubles autistiques), sa réfraction (par ex. sous la forme de données sur sa puissance optique de correction), son port de lunettes ou lentilles de contact.

Les protections qui seront apportées par le filtre de lumière recherché visent à améliorer ou à maintenir son confort visuel (par ex.: absence d'éblouissement) ou bien ses performances visuelles (par ex. son acuité visuelle).

Par maintenir les performances visuelles, on entend également la protection de l'œil vis-à-vis de rayonnements nocifs tels que le rayonnement ultra-violet (UV), la lumière bleue photo-toxique, ou bien le rayonnement infra-rouge (IR).

Les données usages et/ou besoins permettent de présélectionner des filtres d'essai potentiellement intéressants pour l'individu.

Par exemple, si l'individu pour lequel on recherche un filtre de lumière se plaint régulièrement de problèmes d'éblouissement, on évitera de sélectionner des verres faiblement teintés qui *a priori* ne permettront certainement pas d'apporter une solution satisfaisante en termes de confort visuel
On verra également que les données collectées vont permettre de déterminer les exigences visuelles de l'individu et d'aider à paramétrer les scènes lumineuses.

Par exemple, si le sujet relate une gêne accrue pour la conduite de nuit spécifiquement, on choisira des scènes lumineuses avec :
- un environnement lumineux de type mésopique, voire scotopique ;
- des sources lumineuses ponctuelles (ex.: phares de voiture, lampadaire, etc...) ; et/ou
- une scène de route, avec des piétons ou obstacles spécifiques, avec des tests de reconnaissance d'objet ou de contraste et/ou de temps de réaction.

Une mesure du champ attentionnel de l'individu pourra aussi être implémentée pour évaluer le risque d'accident et évaluer le bénéfice du filtre sur ces mesures qualitatives et quantitatives.

Dans un mode de réalisation particulier, la collecte des données se fait au moyen d'un questionnaire.

On soumet alors à l'étape a) l'individu à ce questionnaire, les données collectées comprenant les réponses aux différentes questions du questionnaire.

Le questionnaire peut être réalisé au moyen d'un support papier ou bien d'un support numérique (par exemple : ordinateur, tablette, ou smartphone). Alternativement, le questionnaire peut se faire sous forme orale avec un praticien qui pose les questions oralement et note les réponses, soit sur un support papier, soit sur un support numérique.

Les réponses à certaines questions peuvent être binaires (oui/non), ou bien du type « jamais/parfois/souvent/toujours ». Parfois, les réponses peuvent être une note, par exemple comprise entre 1 et 5.

Dans un autre mode de réalisation particulier, on recueille à l'étape a), en plus ou à la place du questionnaire, des mesures relatives à l'environnement lumineux de l'individu au moyen d'au moins un capteur de lumière, les données collectées étant fonction de ces valeurs de mesure.

L'étape a) peut alors comprendre une mesure du flux lumineux auquel est habituellement soumis le porteur. Elle est réalisée à l'aide d'un capteur de flux lumineux indépendant ou intégré à une paire de lunettes ou un objet connecté du porteur, par exemple un smartphone, une tablette ou une montre connectée qui recueillent les caractéristiques du flux lumineux ambiant à cet instant présent. Ce capteur (type spectrophotomètre) permet de recueillir les caractéristiques du flux lumineux auquel est soumis le porteur pendant qu'il remplit le questionnaire (notamment intensité, spectre, variation dans le temps).

L'idée d'une mesure connectée est de mesurer les habitudes d'exposition à la lumière (intensité, spectre, variations d'intensité) d'un individu dans son environnement habituel et de les associer à un degré d'éblouissement par exemple.

Les données collectées permettent d'orienter les paramètres du filtre d'essai selon le type et la fréquence d'éblouissement du porteur.

On peut ainsi considérer un filtre d'essai avec une transmission lumineuse minimale à prescrire selon le niveau d'intensité lumineuse à partir duquel l'individu est gêné.

Aussi, si l'individu montre un inconfort visuel plus important dans les variations de lumière, on peut orienter la sélection du filtre d'essai vers un filtre actif avec une transmission visuelle dynamique, contrairement à un filtre passif.

Dans une première version, on peut prévoir une application dédiée sur le smartphone de l'individu permettant de récupérer des données à chaque fois que le smartphone est utilisé. Dans une première version améliorée, les données sont collectées en continu.

Les données enregistrées par le capteur de lumière (par ex. le capteur photographique du smartphone) peuvent être : la luminosité ambiante, la date, la localisation (position donnée par le capteur GPS du smartphone), les données météo et la gêne du sujet liée à l'éblouissement (*via* une question ouverte sur le degré d'éblouissement avec une réponse sur une échelle de 1 à 5).

Avantageusement, on peut également prévoir que le capteur photo enregistre une image ou un film de l'environnement lumineux où se trouve l'individu au moment de ses réponses. Les données ainsi récupérées permettent alors d'associer une gêne à une luminosité.

Dans une seconde version, on peut prévoir d'utiliser, en plus du smartphone de l'individu, des lunettes équipées d'un capteur de luminosité que l'individu porte lorsqu'il répond au questionnaire.

La luminosité ambiante est enregistrée en continu et les informations de localisation, ainsi que les indicateurs d'éblouissement, ... sont enregistrées à intervalles réguliers *via* le smartphone.

L'ensemble des données en lien avec les besoins visuels et usages de l'individu déterminera les scènes lumineuses subies par l'individu à l'étape c) du procédé (bloc 31 de la figure 1).

Il définira également les exigences visuelles du porteur.

Dans un mode de réalisation préféré, ces éléments pourront alors déterminer les critères d'évaluation du filtre (étape b1 ci-dessous), voire donner des orientations sur les paramètres du filtre à sélectionner.

### Étape b1 (bloc 21 de la figure 1)

Cette étape de sélection b1) consiste à sélectionner au moins un filtre d'essai sur la base d'au moins un critère prédéfini d'évaluation.

Dans le mode de réalisation de l'invention décrit ici, le ou les filtres d'essai sont sélectionnés en fonction des données collectées à l'étape a (bloc 11).

En variante, on peut prévoir que le filtre d'essai soit sélectionné parmi un ensemble de filtres d'essai prédéterminés.

L'objectif est de définir des critères d'évaluation du filtre d'essai qui sont basés soit sur des tests génériques soit sur le type d'environnement ou des besoins identifiés par l'individu.

Les critères d'évaluation peuvent être objectifs, subjectifs ou mixtes.

Par exemple, le confort visuel, la perception des couleurs, ou la fatigue oculaire peuvent être notés sur une échelle calibrée de 1 (confort mauvais/perception mauvaise des couleurs/fatigue importante) à 5 (confort excellent/très bonne perception des couleurs/ fatigue faible).

Le ou les critères d'évaluation ne faisant pas partie de l'invention le résultat d'une mesure de performance visuelle appliquée à des tests psycho-physiques :
- sensibilité aux contrastes, perception du mouvement ou de la profondeur ;
- performance/vitesse de lecture ;
- reconnaissance d'objet dans des scènes visuelles, mesure de temps de réaction lors de situation de conduite ;
   - mesure du seuil de gêne / de sensibilité à la lumière ;

Enfin, les critères d'évaluation peuvent être objectifs et concerner des données physiologiques en lien avec les performances visuelles et/ou le confort visuel telles que, par exemple : dynamique de la pupille, mouvements des paupières, signal électro-physiologique provenant de la rétine et/ou du cortex cérébral (mesurables *via* électro-encéphalogramme (EEG), électro-rétinographie de l'œil (ERG), ou potentiels évoqués visuels (PEV)).

Le filtre d'essai peut être défini par au moins l'un des paramètres suivants :
- valeur de Tᵥ: pourcentage de transmission visuelle dans le domaine visible en condition photopique telle que définie dans la norme ISO 8980-3. Il est défini dans la gamme de longueurs d'onde allant de 380 nm à 780 nm comme la moyenne pondérée par la sensibilité standard V(λ) en condition photopique de l'œil sous un illuminant D65 (lumière du jour). La transmission visuelle Tv en condition scotopique peut être définie de la même manière avec la sensibilité standard V'(λ) en condition scotopique ;
- réponse spectrale : par ex. donnée de la transmission lumineuse en fonction de la longueur d'onde, dans le domaine visible entre 380 nm et 780 nm, et/ou dans le domaine ultra-violet, et/ou dans le domaine infra-rouge ;
- variation spatiale (par ex. gradient) du facteur de réflexion ou d'absorption d'un verre en présence du filtre d'essai (filtre « dégradé ») ;
- variation temporelle de l'un des paramètres ci-dessus (par ex. filtre photo-chromique ou électro-chromique) ; ou
- variation de l'état de polarisation de la lumière transmise.

Les paramètres du filtre d'essai pourront être pris les uns après les autres et testés au fur et à mesure selon les critères d'évaluation.

Ils sont présélectionnés selon la collecte de données de l'étape a (bloc 11 de la figure 1).

Par exemple, si le seuil de sensibilité à la lumière de l'individu est important, on ne testera pas des filtres d'essai de classe 0 ou 1 au sens de la norme NF EN ISO 12312-1, mais on commencera avec des filtres avec une transmission visuelle (Tᵥ) plus protecteur.

Aussi, selon les environnements lumineux de l'individu, on pourra présélectionner certains spectres pour lequel l'individu ressent plus de gêne.

Les paramètres des différents filtres d'essai à sélectionner peuvent également être sélectionnés selon une procédure pré-établie, par exemple en choisissant d'abord la transmission visuelle Tᵥ, puis en raffinant la sélection en fonction des réponses spectrales, et enfin en orientant ou non vers une adaptation photo-chromique ou électro-chromique du/des filtre(s) d'essai (si sensibilité dynamique à la lumière par exemple).

### Étape b2) (bloc 22 de la figure 1)

Cette étape de sélection b2) (qui peut avoir lieu avant ou après l'étape de sélection b1) consiste à sélectionner, en fonction des données collectées à l'étape a (bloc 11) au moins une scène lumineuse qui est représentative des usages et/ou besoins visuels de l'individu testé.

Cette scène lumineuse servira pour la mise en situation de l'étape c (bloc 31 de la figure 1).

De manière avantageuse, cette scène lumineuse comprend, d'une part, une image, ou un film, déterminée en fonction de ces besoins et usages, et, d'autre part, au moins une source lumineuse génératrice d'un inconfort et/ou d'une perte de performances visuelles pour l'individu en l'absence de port d'un filtre.

De préférence, l'image (ou le film) de la scène visuelle est définie à la fois par au moins un des deux paramètres suivants :
- un paramètre physique ou optique des conditions lumineuses : intensité (cd), luminance (cd/m²), éclairement (lux), spectre en fonction de la longueur d'onde, variation temporelle de ces paramètres, localisation et orientation de la source (source ponctuelle ou diffuse), etc... ;
- un paramètre relatif aux objets ou aux activités visuelles à implémenter (par ex. : situation de conduite automobile, marche, lecture sur terrasse, perception de panneaux ; simple stimulus visuel comme une lettre d'acuité visuelle, une cible en mouvement ou colorée, stimulus central vs. périphérique, ... ; aucun stimulus visuel).

L'ensemble de ces paramètres peut ainsi représenter la richesse et la complexité des situations lumineuses auxquelles l'individu risque d'être confronté.

En d'autres termes, l'image (ou le film) de la scène lumineuse est un ensemble d'objets, représentatifs d'une situation de vie réelle de l'individu, caractérisée par des objets, définis par leurs positions, leurs tailles et leurs formes. Pour chaque objet, l'environnement lumineux est défini (intensité, spectre, distribution spatiale et variation temporelle).

Ces éléments de l'environnement vont permettre de tester l'individu dans des situations proches de ses besoins et également orienter les critères d'évaluation des filtres d'essai.

Selon certains modes de réalisation, l'image ou le film comprend des stimuli visuels standards, tels que ceux utilisés lors des tests optométriques classiques : lettre d'acuité visuelle de 1/20^{ème} à 20/10^{ème} avec un contraste entre 5% et 100%, mires de contraste allant de 1% à 100% avec différentes fréquences spatiales, motifs pour la vision des couleurs ou de la profondeur, film pour la perception des mouvements ou bien film pour la simulation d'une situation de marche ou de conduite automobile, champ visuel attentionnel, détection de patch.

Afin de valider le filtre d'essai quant à sa capacité à améliorer le confort et/ou les performances visuelles, la scène lumineuse sélectionnée peut comprendre, et, de préférence, comprend également une ou plusieurs sources lumineuses, chaque source lumineuse étant génératrice d'un inconfort et/ou d'une perte de performances visuelles pour l'individu lorsque celui-ci ne porte pas de filtre.

La ou les sources lumineuses de la scène viennent donc se superposer ou s'ajouter à l'image ou au film précédemment décrit.

Avantageusement, on prévoit de pouvoir sélectionner un très grand nombre de sources lumineuses en fonction des besoins et usages recueillis à l'étape a) :
- source ponctuelle ou quasi ponctuelle, source étendue ;
- source directionnelle ou diffuse ;
- source plus ou moins intense (niveau de luminance ou de flux lumineux) ;
- source colorée ou non (i.e. sensiblement blanche), source à T° de couleur froide ou chaude ;
- source continue ou intermittente/transitoire, avec variation temporelle de ses paramètres (intensité, direction d'émission, spectre, ...) ;
- source primaire ou secondaire ;
- source naturelle ou artificielle ; ou
- source provenant d'une lampe à incandescence, halogène, à décharge, à vapeur (de sodium par exemple), source de type diode électroluminescente (LED), éventuellement organique (OLED), ....

De manière générale, la source lumineuse additionnelle dans l'image émet un « *flux* » lumineux additionnel pour l'individu observant la scène lumineuse.

De préférence, on prévoira des sources lumineuses pouvant générer un flux lumineux additionnel de sorte que la luminance visuelle de l'ensemble formé par la scène lumineuse et la source lumineuse additionnelle présente une luminance visuelle :
- supérieure à 60 cd/m², de préférence supérieure ou égale à l'une des valeurs suivante : 300 cd/m², 500 cd/m², 1000 cd/m², 1800 cd/m², lorsque l'image de la scène lumineuse observée seule par l'individu le place dans des conditions photopiques de vision (luminance moyenne de l'image ou du film supérieure à 10 cd/m²) ; et
- supérieure à 1 cd/m², dans des conditions mésopiques de vision (luminance moyenne de l'image ou du film comprise entre 10⁻³ et 10 cd/m²) ; et
- supérieure à 10⁻³ cd/m² dans des conditions scotopiques de vision (luminance moyenne de l'image ou du film inférieure à 10⁻³ cd/m²).

En conditions photopiques, on prévoit aussi de faire varier le flux lumineux additionnel de la source lumineuse de sorte que l'éclairement dans le plan de l'œil soit dans un mode de réalisation d'au moins 200 lux (Ix), de préférence supérieure ou égale à l'une des valeurs suivantes : 500 Ix, 1000 lx, 1500 Ix, 2000 lx, 5000 lx, 10000 lx, mieux 15000 lx. Ces gammes d'éclairement correspondent aux valeurs d'éclairement reçus par l'œil dans un environnement naturel en journée par temps couvert à lumineux.

De préférence encore, on prévoit de pouvoir faire varier le spectre, la couleur, et/ou la température de couleur de la source lumineuse additionnelle, le spectre pouvant être étroit ou large, discret ou continu, mono- ou polychromatique.

Avantageusement, le niveau et/ou le spectre du flux lumineux additionnel peuvent être programmés en choisissant les différentes sources de lumières (par ex. différentes LED), ou par le port de lunettes à verres passifs ou actifs (électrochrome) dont la teinte, la réponse spectrale, et/ou la vitesse d'activation peuvent être contrôlées.

### Étape c (bloc 31 de la figure 1)

Une fois que la scène lumineuse et le filtre d'essai ont été sélectionnés en fonction des besoins et usages de l'individu récoltés à l'étape a), il est prévu une étape de mise en situation de l'individu lors de laquelle on lui présente visuellement la scène lumineuse au travers du filtre d'essai choisi.

On remarquera tout d'abord que le filtre d'essai peut être réel et/ou virtuel.

Lorsque le filtre d'essai est réel, alors à l'étape c), l'individu porte réellement, c'est-à-dire physiquement, ce filtre d'essai pour visionner la scène lumineuse qui est affichée sur un dispositif d'affichage observé par l'individu.

De cette façon, il est possible pour l'individu de se rendre compte quel est l'impact du filtre d'essai réel sur la scène lumineuse affichée, en particulier sur son confort visuel et/ou ses performances visuelles.

Comme il sera détaillé plus avant dans la suite de la description, le dispositif d'affichage utilisé lors de l'étape c) est de préférence adapté pour afficher tous les types de scènes lumineuses précédemment décrites, et en particulier les scènes lumineuses dans lesquelles la source lumineuse additionnelle génère un éblouissement de l'individu.

Pour cela, on peut avantageusement prévoir d'utiliser des scènes lumineuses à haute dynamique et un dispositif d'affichage comportant un écran graphique HDR (*High Dynamical Range*) capable d'afficher ce type de scènes lumineuses.

Un filtre d'essai réel peut être par exemple réalisé sous la forme d'un aplat coloré rapporté sur une lentille ophtalmique ou une lunette d'essai ou bien sous la forme d'un verre teinté.

Un autre type de filtre réel peut être formé par un verre actif, de type électrochrome ou bien à cristaux liquides.

Le filtre d'essai peut donc également être virtuel, c'est-à-dire non physique. On entend ici que le filtre d'essai, ou plus précisément l'effet du filtre d'essai, est reproduit par simulation.

En pratique, la simulation est faite en calculant les modifications induites par le filtre d'essai virtuel (ses caractéristiques optiques) sur la scène visuelle (distribution de luminance, contenu spectral, variation temporelle, ...) avant la présentation de la scène lumineuse à l'individu.

En d'autres termes, à l'étape c), la scène lumineuse est simulée et affichée par le dispositif d'affichage au travers du filtre d'essai sélectionné telle qu'elle serait vue par l'individu s'il portait réellement ledit filtre d'essai.

Ainsi, lorsque l'individu observe le dispositif d'affichage, il perçoit l'effet du filtre directement sur la scène lumineuse rendue de manière réaliste sur le dispositif d'affichage visionné par l'individu.

Grâce à cette possibilité de simulation des effets du filtre d'essai sur la scène lumineuse sélectionnée en fonction des besoins et usages de l'individu, il est possible de tester un grand nombre de filtres d'essai, et de faire varier facilement les caractéristiques optiques du filtre d'essai.

Par exemple, si le filtre d'essai est un filtre solaire sous la forme d'un verre teinté, de couleur légèrement verte, le bénéfice de ce filtre d'essai sur le confort visuel de l'individu, par exemple sa sensibilité à l'éblouissement, sera testé lors de la mise en situation de l'individu à l'étape c) en calculant la modification du contenu spectral de la scène lumineuse par la réponse spectrale du filtre.

Avantageusement, on peut également prévoir avant l'étape c) une calibration du dispositif d'affichage pour caractériser les propriétés de son écran graphique en termes photométriques et visuels : luminance maximale de l'écran (en cd/m²), son gamma par canal, sa température de couleur (en kelvins) ou sa valeur de chrominance du point blanc, son contraste statique ou dynamique, ses caractéristiques spectrales, etc....

Bien évidemment, il est possible lors de l'étape c) de mettre en situation l'individu avec deux filtres d'essai : un filtre réel et un filtre virtuel. Pour cela, il suffit que l'individu observe le dispositif d'affichage au travers du filtre d'essai réel, le dispositif d'affichage simulant et affichant la scène lumineuse telle qu'elle serait vue si l'individu ne portait que le filtre d'essai virtuel.

### Étape d (bloc 41 de la figure 1)

Pendant ou après l'étape c) de mise en situation, le procédé de détermination comporte une étape d'évaluation lors de laquelle on *« note »* le filtre d'essai sélectionné pour chacun des critères préfinis d'évaluation.

L'évaluation du bénéfice du filtre d'essai sélectionné comprend la mesure de l'acuité visuelle.

Elle peut également être faite soit directement par l'individu qui note lui-même un critère prédéfini (par ex. : l'individu donne une note comprise entre 1 et 5 pour son appréciation des couleurs avec le filtre d'essai choisi), ou indirectement par l'individu qui effectue un test, le résultat de ce test constituant la note donnée au critère prédéfini ( résultat d'un test d'acuité visuelle).

Pour chaque critère prédéfini, on enregistre le résultat de l'évaluation indirecte. Ne faisant pas partie de l'invention, le résultat de l'étape d) peut également être une valeur pondérée des différentes notes obtenues pour le filtre d'essai en test en fonction de différents critères prédéfinis.

La pondération peut être une simple moyenne (chaque critère a le même poids) ou une pondération plus compliqué en fonction de l'importance des besoins exprimés par l'individu.

### Étape e (bloc 51 de la figure 1)

Une fois que le ou les résultats de l'étape d) d'évaluation ont été obtenus, on compare chacun à un seuil prédéterminé d'acuité visuelle.

Ce seuil prédéterminé d'acuité visuelle peut être un seuil absolu ou bien un seuil relatif.

Par exemple, si le critère prédéfini d'évaluation du filtre d'essai à tester concerne sa capacité à restaurer une acuité visuelle satisfaisante en condition d'éblouissement, on peut estimer que le filtre d'essai testé présente un bénéfice pour l'individu si l'acuité visuelle mesurée (i.e. le résultat de l'étape d d'évaluation) est supérieure ou égale à un seuil absolu de 7/10^{ème}.

Inversement, on peut vouloir comparer à cette étape le bénéfice du filtre d'essai par rapport à une situation où l'individu ne porte aucun filtre d'essai ou bien une situation où l'individu porte un autre filtre qui peut être soit un filtre d'essai de référence ou bien simplement le filtre d'essai précédemment testé. On peut ainsi avoir une démarche incrémentale où l'on cherche une amélioration du confort visuel ou des performances visuelles relativement à un autre filtre d'essai déjà testé sur l'individu.

### Étape f (bloc 61 de la figure 1)

La validation ou non de la prescription du filtre de lumière est réalisée sur la base du résultat de la comparaison de l'étape e).

On peut rechercher une validation ciblée (comparaison avec un seuil absolu) ou bien une validation relative (comparaison avec un seuil relatif) du filtre d'essai.

Si la comparaison de l'étape e) montre une amélioration du critère prédéfini, alors l'étape f), ledit filtre d'essai est retenu comme étant ledit filtre de lumière adapté pour l'individu.

À l'inverse, si la comparaison de l'étape e) montre une dégradation du critère prédéfini, alors à l'étape f), on reprend le procédé de détermination à l'étape b1) en sélectionnant un autre filtre d'essai (voir flèche en trait pointillé entre le bloc 61 et le bloc 21 de la figure 1) puis en répétant les étapes c), d), e) et f) avec ce nouveau filtre d'essai. L'optimisation du filtre peut donc être itérative.

Toutefois, l'itération peut être réalisée même si la comparaison montre une amélioration du critère prédéfini, de façon à tendre vers un filtre de lumière optimal.

On peut imaginer introduire une certaine tolérance dans la comparaison de l'étape e), de sorte qu'un filtre d'essai est considéré comme validé, respectivement invalidé, dès que l'amélioration, respectivement la dégradation, est supérieure en valeur absolue à une valeur de tolérance ε prédéterminée, fonction notamment de la précision de mesure.

Ceci évite de réaliser trop d'itérations pour un critère prédéfini donné et de converger rapidement vers un filtre de lumière optimal.

À la fin du procédé, on a donc déterminé un filtre de lumière qui est adapté aux besoins et usages de l'individu collectés à l'initialisation du procédé.

### EXEMPLES

On décrit ci-dessous deux exemples d'application du procédé de détermination tel que décrit ci-dessus.

### Exemple 1

On cherche à déterminer un filtre de lumière pour un individu qui a une acuité visuelle :
- de 9/10^{ème} lorsqu'il est muni de son équipement de correction visuelle (lunettes correctrices) ; et
- de 6/10^{ème} en condition d'éblouissement, par exemple sous fort soleil.

On peut grâce au procédé tester une série prédéterminée de filtres d'essai sur l'individu, par exemple en rapportant ces filtres sur chacun des verres de ses lunettes. Les différents filtres d'essai ont des valeurs de transmission visuelle Tv et des réponses spectrales (atténuation en fonction de la longueur d'onde par exemple) différentes.

Grâce au dispositif d'affichage, on simule à l'étape c) une condition lumineuse au-delà du seuil de sensibilité de l'individu (valeur préalablement mesurée).

On mesure alors pour chaque filtre d'essai l'acuité visuelle de l'individu. On sélectionne le ou les filtres d'essai qui permettent de restaurer une acuité visuelle d'au moins 9/10^{ème} en environnement lumineux.

### Exemple 2 (ne faisant pas partie de l'invention)

Un individu ayant une pathologie visuelle est très sensible à la lumière et sa performance visuelle est très sensible à l'effet d'un filtre de lumière. L'individu peut avoir besoin d'un filtre de lumière même en condition de faible luminosité (situation en intérieur par exemple), car le filtre de lumière permet alors de rehausser d'autres fonctions visuelles et améliorer le confort.

Dans ce cas de figure, on sélectionne à l'étape b1) des filtres d'essai selon leur réponse spectrale dans le domaine visible (380-780 nm).

En fonction des besoins spécifiques de l'individu (qui dépendent de sa pathologie et de sa sensibilité propre), on sélectionnera des filtres d'essai de réponses spectrales différentes et on les évaluera selon les critères prédéfinis suivants :
A) amélioration de la netteté ;
B) apport de luminosité ;
C) altération des couleurs ;
D) protection à la lumière.

Le tableau ci-après récapitule des résultats obtenus grâce au procédé de détermination de l'invention.

Ce tableau oriente le premier choix de filtre d'essai (spectre) préféré, par gamme de teinte, selon les critères d'une population d'individus, que l'on testera en premier.

Par exemple, si l'individu privilégie le critère A « netteté » en premier, on testera en premier un filtre spectral à 450 nm (filtre F11) et on évaluera l'évolution du critère netteté sur une image statique ou dynamique.

Si le sujet privilégie la protection à la lumière en condition de luminosité intérieure, on testera le filtre n°F15 de préférence, pour le comparer ensuite à d'autres filtres.

| **Critère A** | **Critère B** | **Critère C** | **Critère D** |
|---|---|---|---|
| Filtre F11 | Filtres F11, F12, F13 | Filtres F12, F14 | Filtre F15 |
| Si 2^{ème} critère = C : filtres F22, F24 | | | Si 2^{ème} critère = A : F21, F'21 |
| Si 2^{ème} critère = D : filtres F21, F22 | | | |

### Dispositif

Sur la figure 2, on a représenté un casque 70 de réalité virtuelle destiné à être porté par un individu pour lequel on cherche à déterminer un filtre de lumière optimal afin de restaurer ou de maintenir son confort visuel et/ou ses performances visuelles.

Ce casque 70 de réalité virtuelle est ainsi particulièrement adapté pour mettre en œuvre le procédé de détermination de l'invention décrit ci-dessus.

Globalement, ce casque 70 est un casque classique de réalité virtuelle auquel on a ajouté une ou plusieurs sources lumineuses permettant de simuler des objets ou des sources de luminosité élevée à même de générer un éblouissement pour un individu.

Dans ce cas, l'individu est immergé dans un environnement lumineux très réaliste, qui permet de simuler des scènes lumineuses de la vie quotidienne, de simuler les mouvements de la scène lumineuses ou des sources éblouissantes de manière réaliste et donc de permettre à l'individu de faire un retour rapide et fiable sur son niveau d'inconfort visuel ou de performance visuelle.

Comme le montre bien la figure 2, le casque 70 de réalité virtuelle comporte tout d'abord un dispositif d'affichage 80 destiné à tester le confort visuel et/ou les performances visuelles de l'individu et comportant à cet effet un écran graphique 91 et un système d'illumination additionnel 100.

L'écran graphique 91 est, dans le mode de réalisation présenté ici, l'écran d'affichage d'un smartphone 90. Il est ici commandé par le micro-processeur (non représenté) du smartphone 90 pour afficher sur cet écran graphique 91 une image (ou un film) susceptible d'être visionnée par l'individu testé qui porte le casque 70.

On entend ici que l'image (ou le film) est une image (ou un film) générée de la scène lumineuse sélectionnée lors de l'étape de sélection du procédé de détermination de l'invention.

En variante, l'écran graphique peut être un écran dédié du casque de réalité virtuelle et fourni avec celui-ci. Dans ce cas, l'écran graphique peut être commandé par un micro-processeur spécifique du casque de réalité virtuelle.

Dans un mode de réalisation préféré, le micro-processeur commande l'écran graphique 91 de manière à ce que cet écran graphique 91 affiche une image (ou un film) formée d'une image (film) gauche pour l'œil gauche 2 et d'une image (film) droite pour l'œil droit 1 de l'individu.

Dans ce mode de réalisation, la casque 70 comporte également des moyens optiques de visualisation tridimensionnelle adaptés pour présenter l'image gauche, respectivement l'image droite, à l'œil gauche de l'individu, respectivement à l'œil droit de l'individu, de sorte que celui-ci visualise une image (ou un film) tridimensionnelle par fusion des images (films) gauche et droite.

Les moyens optiques de visualisation tridimensionnelle comprennent ici (voir fig. 2, 5, 6, et 7) deux lentilles, une lentille droite 71 et une lentille gauche 72, placées respectivement devant l'œil droit 1 et l'œil gauche 2 de l'individu et positionnées de manière à ce que l'individu voie l'image droite et l'image gauche affichées sur l'écran graphique 91 du smartphone 90.

De manière avantageuse, on peut prévoir que les deux lentilles 71, 72 forment des images intermédiaires à partir des images gauche et droite, ces images intermédiaires étant formées dans un plan bien déterminé, par exemple dans un plan situé à l'infini optiquement.

Ce plan peut également être à une distance de vision proche, vision intermédiaire ou vision de loin, ou à toute autre distance, selon l'amétropie du porteur et/ou selon la volonté de présenter une scène à une distance particulière pour le test.

Le système lumineux additionnel 100 est quant à lui adapté pour diffuser, à l'individu observant l'écran graphique 91, un flux lumineux additionnel (voir flèches F1, F2 sur la figure 2). L'individu reçoit donc le flux lumineux provenant des images affichées sur l'écran graphique 91 et le flux lumineux additionnel F1, F2 émis par le système d'illumination additionnel 100.

À cet effet, le casque 70 comporte des moyens 74, 75, 79 pour maintenir le dispositif d'affichage 80 devant les yeux 1, 2 de l'individu. En particulier, il est prévu, d'une part, des fixations 79 permettant d'attacher le dispositif d'affichage 80 au boîtier 74 du casque 70 et, d'autre part, une attache 75 permettant à l'individu de fixer le casque 70 sur sa tête, l'attache 75 venant sertir son crâne.

Le dispositif d'affichage 80 est fixé au casque 70 grâce aux fixations 79 de sorte que l'écran graphique 91 du smartphone 90 est tourné vers les yeux 1, 2 de l'individu et que le dispositif d'illumination additionnel 100 est agencé par rapport à cet écran graphique 91 pour s'interposer entre celui-ci et les yeux 1, 2 de l'individu.

On verra dans la suite de la description que le système lumineux additionnel 100 est conçu pour laisser visible tout ou partie de l'image ou du film affiché sur l'écran graphique 91 du smartphone.

Le casque 70 de réalité virtuelle comporte également une jupe 73 adaptée à être plaquée sur la face de l'individu lorsque celui-ci porte le casque 70 avec la lanière élastique 75 serrée contre l'arrière de son crâne.

Cette jupe 73 est opaque et permet alors d'isoler l'individu de la lumière ambiante régnant autour de l'individu.

De cette façon, lors de la mise en œuvre du procédé de détermination, l'individu est placé dans des conditions lumineuses maîtrisées, qui ne dépendent que de la lumière diffusée par l'écran graphique 91 (images gauche et droite) et le dispositif d'illumination additionnel 100 (flux lumineux additionnel F1, F2).

Le système d'illumination additionnel 100 est commandé par un autre micro-processeur ou, comme ici, par le micro-processeur du smartphone 90. Il est alors prévu un élément d'interfaçage entre ce micro-processeur et le système d'illumination additionnel 100 permettant à celui-ci de recevoir et traiter des instructions de la part du micro-processeur.

Le micro-processeur du smartphone 90 commande le dispositif d'illumination additionnel 100 pour que celui-ci émette un flux lumineux additionnel F1, F2 présentant une énergie, une répartition spectrale, une variation temporelle, une distribution spatiale, et/ou une distribution angulaire prédéterminée(s). On verra dans la suite de la description différents mode de réalisation du système d'illumination additionnel 100.

De préférence, le système d'illumination additionnel 100 diffuse un flux lumineux additionnel F1, F2 permettant d'atteindre une luminance visuelle supérieure ou égale à 1000 candelas par mètre-carré (cd/m²), de préférence supérieure ou égale à 2000 cd/m², mieux supérieure ou égale à 3000 candelas par mètre-carré (cd/m²) et de préférence jusqu'à 20000 cd/m².

Un tel niveau de luminance visuelle rend possible la génération d'environnements lumineux dans lesquels le flux lumineux additionnel F1, F2 est susceptible de générer un inconfort visuel de l'individu, en particulier par éblouissement.

Avantageusement, le dispositif d'affichage 80 comporte deux capteurs de lumière 77, 78 (voir figure 2) adaptés à délivrer un signal représentatif du niveau d'éclairement (lux) au niveau des deux yeux 1, 2 de l'individu, et dans lequel le niveau moyen de luminance de l'image (ou du film) et/ou du flux lumineux additionnel F1, F2 est commandé en fonction de ce signal représentatif.

On peut également prévoir des moyens de fixation 109, par exemple par clipsage, du système d'illumination additionnel 100 sur le corps du smartphone 90.

Comme expliqué ci-dessus, le système d'illumination additionnel 100 est agencé par rapport à l'écran graphique 91 de manière à laisser visible tout ou partie de l'image qu'il affiche.

Pour cela, dans le premier mode de réalisation représenté sur la figure 2, le dispositif d'affichage 80 comporte un système d'illumination additionnel 100 comprenant ici deux séries 101, 102 de diodes électroluminescentes (LEDs) blanches pilotables indépendamment et disposées sur la tranche d'une dalle 111 en matériau plastique transparent, du type polycarbonate ou PMMA par exemple, jouant le rôle de guide d'onde optique pour la lumière des diodes électroluminescentes 101, 102 couplée à l'intérieur de la dalle 111.

Le système lumineux additionnel comprend également un film diffusant 110 actif ou passif déposé sur toute ou partie de la face arrière 112 de la dalle lumineuse 111 tournée vers les yeux 1, 2 de l'individu et adapté à diffuser la lumière guidée par réflexion totale interne dans la dalle 111 lumineuse.

Avantageusement, ce film diffusant 110 peut être un film actif de type à cristaux liquides polymère dispersé (film PDLC pour *Polymer-Disperse Liquid Crystal*) comprenant des zones activables permettant de diffuser ou non la lumière.

On a représenté sur les figures 3 et 4 un exemple de film diffusant actif pouvant être utilisé dans le système d'illumination additionnel 100 de l'invention.

Dans ces deux figures, le diffuseur 110 est formé d'un film diffusant de type PDLC, de 120 microns d'épaisseur, commercialisé par la société Kyushu Nanotec Optics. Ce film actif comporte deux états : un état diffusant à l'état OFF et un état transparent à l'état ON.

Le film diffusant 110 comporte, pour chaque œil droit 1 et gauche 2 et situées de chaque côté d'un axe central 76 du casque 70, deux zones activables 112, 113 et 114, 115 :
- une zone activable centrale 113 (œil droit), 115 (œil gauche) ; et
- une zone activable périphérique 112 (œil droit), 114 (œil gauche).

Ces zones activables 112, 113, 114, 115 sont commandées par le micro-processeur du smartphone 90 pour être diffusantes (état « OFF ») ou transparentes (état « ON ») en fonction du contenu affiché sur l'écran graphique 91.

Avantageusement, ces zones activables 112, 113, 114, 115 peuvent être, par exemple, deux ouvertures permettant la vision stéréoscopique avec l'œil droit et gauche 1, 2 des images ou du film affiché sur l'écran graphique.

Un pilotage indépendant de ces deux zones permet de réaliser des tests de vision indépendamment sur l'œil droit 1 ou gauche 2 ou sur les deux. L'avantage d'utiliser une zone centrale et une zone périphérique est de pouvoir ajuster la taille de la zone centrale en fonction de la dimension des scènes lumineuses à observer. Les zones peuvent donc être de dimensions multiples.

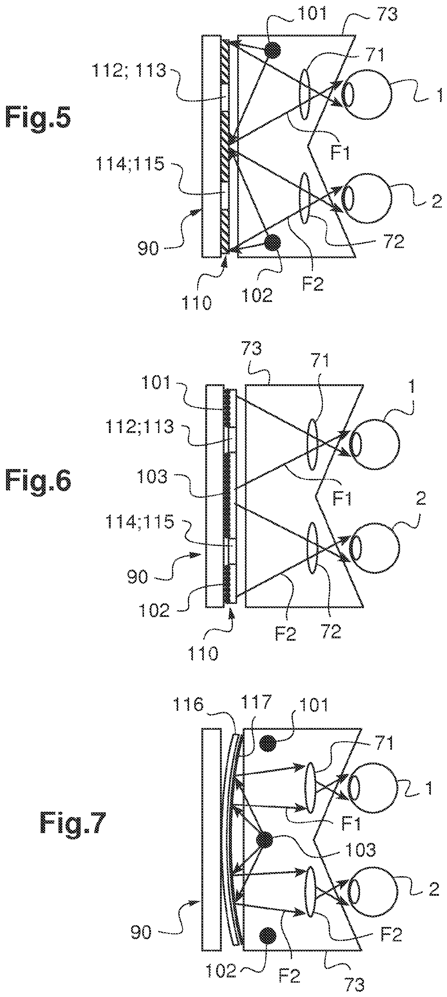

Les figures 5, 6, et 7 représentent respectivement des vues schématiques d'un dispositif d'affichage selon un deuxième mode de sation et un troisième et quatrième exemples ne faisant pas partie de l'invention.

Dans le deuxième mode de réalisation représenté sur la figure 5, le système d'illumination additionnel comprend deux groupes 101, 102 de diodes électro-luminescentes, ici d'une diode électroluminescente, et un film diffusant 110 actif avec les zones activables 112, 113 et 114, 115 précédemment décrites.

Ce film diffusant 110 s'interpose entre les yeux 1, 2 de l'individu et l'écran graphique 91 du smartphones 90, et rétrodiffuse vers l'individu le flux lumineux émis par les diodes électroluminescentes 101, 102.

Dans le troisième exemple représenté sur la figure 6, le système d'illumination additionnel 100 comprend une dalle lumineuse formée ici d'une matrice de diodes électroluminescentes 101, 102, 103 placée devant ledit écran graphique, et un diffuseur 110 actif en transmission qui s'interpose entre la dalle lumineuse et les yeux 1, 2 de l'individu.

Dans une variante, les sources lumineuses peuvent comprendre des fibres optiques ou bien des diodes électro-luminescentes organiques (OLEDs).

Dans le quatrième exemple représenté sur la figure 7, le système d'illumination comprend trois diodes électroluminescentes 101, 102, 103 et un miroir semi-réfléchissant 116 s'interposant entre les yeux 1, 2 de l'individu et l'écran graphique 91.

Le miroir semi-réfléchissant 116 réfléchit le flux lumineux émis par les diodes électroluminescentes 101, 102, 103 vers les yeux 1, 2 de l'individu. Plus précisément, le miroir semi-réfléchissant 116 est adapté à projeter sur la cornée ou très proche, dans un plan perpendiculaire à la normale au sommet du miroir semi-réfléchissant 116, ce plan étant quasiment tangent aux sommets des cornées de l'individu.

L'avantage de ce dispositif d'affichage, par rapport aux précédents, est de bénéficier de l'écran graphique 91 tout entier tout en pouvant agir sur les diodes électroluminescentes 101, 102, 103.

Dans d'autres modes de réalisation, on pourrait prévoir un ensemble de commandes ergonomiques permettant à l'individu de faire varier les caractéristiques du flux lumineux additionnel par commande du système lumineux additionnel. On pourrait également enregistrer les données physiologiques de l'individu (niveaux d'éclairement de la source qui créent une gêne de l'individu, temps de recouvrement de la vision nette, recouvrement de la vision stéréoscopique, etc...).

On pourrait prévoir de préenregistrer, dans le smartphone ou l'autre micro-processeur pilotant le système d'illumination additionnel et/ou le film diffusant, un ensemble de protocoles de détermination de filtres de lumière. Ces protocoles pourraient faire évoluer l'intensité lumineuse, les répartitions spectrale, spatiale et temporelle de la ou des sources lumineuses additionnelles.

On pourrait également prévoir un mode « manuel » pour permettre à l'individu de faire varier l'intensité lumineuse du dispositif d'affichage. Dans ce cas, le dispositif d'affichage - ou le casque - peut comprendre des moyens de saisie permettant à l'individu d'exprimer un niveau d'inconfort visuel ressenti par rapport au flux lumineux additionnel généré par le système d'illumination additionnel, et des moyens permettant d'associer l'inconfort visuel à au moins une caractéristique optique (intensité, spectre, distribution angulaire ou spatiale, variation temporelle, etc...) du flux lumineux additionnel généré par le système d'illumination additionnel.

Dans un autre mode de réalisation, on peut prévoir d'utiliser le dispositif d'affichage sans le casque de réalité virtuelle. Dans ce cas, le dispositif d'affichage est alors maintenu par l'individu à une distance typique de lecture. Un ensemble de capteurs de lumière permettra d'ajuster le niveau lumineux en fonction de l'éclairement ambiant. L'écran graphique peut alors comprendre l'écran d'une tablette et le système lumineux additionnel peut comprendre un ensemble de diodes électro-luminescentes placées à la périphérie de l'écran.

Enfin, dans encore d'autres modes de réalisation, le dispositif d'affichage peut être utilisé dans un casque à réalité augmentée ou dans un système de projection.

Dans le cadre d'un casque à réalité augmentée, la scène lumineuse observée peut être la scène lumineuse vue par la caméra du smartphone et affichée sur l'écran de celui-ci, en plus de la partie éblouissante du dispositif d'affichage.

Dans le cadre d'un système par projection, des projecteurs puissants pourraient à la fois servir d'illuminateur et d'afficheur de scène à observer.

La présente invention n'est nullement limitée au mode de réalisation décrit et représenté, mais l'homme du métier saura y apporter toute variante conforme aux revendications.

## Revendications

1. Procédé de détermination d'un filtre de lumière adapté à être appliqué à un verre de lunettes pour améliorer ou maintenir le confort visuel et/ou les performances visuelles d'un individu porteur desdites lunettes, ledit procédé comportant :
a) une étape de collecte (11) de données relatives aux besoins et/ou usages dudit individu ;
b1) une première étape de sélection (21) d'au moins un filtre d'essai sur la base d'au moins un critère d'évaluation, ledit critère d'évaluation étant une mesure de l'acuité visuelle ;
b2) une seconde étape de sélection (22), en fonction desdites données collectées à l'étape a), d'au moins une scène lumineuse comprenant :
- une image ou un film déterminé en fonction des besoins et usages visuels de l'individu ; et
- au moins une source lumineuse génératrice d'un inconfort et/ou d'une perte de performances visuelles pour l'individu non équipé de filtre ;
c) une étape de mise en situation (31) dudit individu lors de laquelle on présente visuellement audit individu ladite scène lumineuse sélectionnée à l'étape b2) au travers dudit filtre d'essai sélectionné à l'étape b1) ;
d) une étape d'évaluation (41) comprenant la mesure dudit critère d'évaluation de l'étape b1) lors de la mise en situation de l'étape c) ;
e) une étape de comparaison (51) du résultat de l'évaluation de l'étape d) avec un seuil prédéterminé d'acuité visuelle ; et
f) une étape de détermination (61) dudit filtre de lumière adapté en fonction du résultat de la comparaison de l'étape e).

2. Procédé de détermination selon la revendication 1, selon lequel, à l'étape b1), ledit filtre d'essai est sélectionné en fonction desdites données collectées à l'étape a).

3. Procédé de détermination selon la revendication 1 ou 2, selon lequel, à l'étape f), ledit filtre d'essai est retenu comme étant ledit filtre de lumière adapté si ladite comparaison de l'étape e) montre une amélioration dudit critère prédéfini.

4. Procédé de détermination selon la revendication 1 ou 2, selon lequel, à l'étape f), si ladite comparaison de l'étape e) montre une dégradation dudit critère prédéfini, on reprend le procédé de détermination à l'étape b1) en sélectionnant un autre filtre d'essai.

5. Procédé de détermination selon l'une des revendications 1 à 4, selon lequel, à l'étape a), on soumet ledit individu à un questionnaire, les données collectées comprenant des réponses audit questionnaire.

6. Procédé de détermination selon l'une des revendications 1 à 5, selon lequel, à l'étape a), on recueille également des mesures relatives à l'environnement lumineux de l'individu au moyen d'au moins un capteur de lumière.

7. Procédé de détermination selon l'une des revendications 1 à 6, selon lequel, à l'étape b1), ledit filtre d'essai est sélectionné d'après l'un des paramètres suivants :
- un niveau de transmission visuelle ;
- un spectre de transmission sur une bande de longueur d'onde donnée ;
- une variation spatiale de ces paramètres sur la surface de ladite lentille ; ou
- une variation temporelle de ces paramètres dans le temps.

8. Procédé de détermination selon l'une des revendications 1 à 7, selon lequel, à l'étape c) :
- ladite scène lumineuse sélectionnée à l'étape b2) est affichée par un dispositif d'affichage ; et
- ledit individu porte réellement ledit filtre d'essai ou bien porte virtuellement ledit filtre d'essai, ladite scène lumineuse étant alors affichée par ledit dispositif d'affichage telle qu'elle serait vue par ledit individu s'il portait réellement ledit filtre d'essai,
- ledit individu observe ledit dispositif d'affichage.

9. Dispositif d'affichage (80) pour déterminer un filtre de lumière optimal afin de restaurer ou de maintenir un confort visuel et/ou des performances visuelles de l'individu, ledit dispositif comportant :
- un écran graphique (91) commandé par un micro-processeur pour afficher sur ledit écran graphique (91) une image ou un film susceptible d'être visionnée par ledit individu testé qui reçoit un flux lumineux provenant de l'image ou du film affichés sur l'écran graphique (91),
- un système d'illumination additionnel (100) commandé par un autre micro-processeur ou par le même micro-processeur pour diffuser, audit individu observant ledit écran graphique (91), un flux lumineux additionnel (F1, F2) présentant une énergie, une répartition spectrale, une variation temporelle, une distribution spatiale, et/ou une distribution angulaire prédéterminée(s), ledit système d'illumination additionnel (100) étant agencé par rapport audit écran graphique (91) de manière à laisser visible tout ou partie de ladite image ou dudit film,
**Caractérisé en ce que** ledit système d'illumination additionnel (100) est adapté à diffuser un flux lumineux additionnel (F1, F2) permettant, en combinaison avec ledit écran graphique (80), d'atteindre une luminance visuelle supérieure ou égale à 1000 candelas par mètre-carré (cd/m²),
dans lequel ledit système d'illumination additionnel (100) comprend des diodes électroluminescentes (101, 102) et un film diffusant (110) actif ou passif s'interposant entre les yeux (1, 2) dudit individu et ledit écran graphique (91), ledit film diffusant (110) étant adapté à rétrodiffuser le flux lumineux émis par lesdites diodes électroluminescentes (101, 102).

10. Dispositif d'affichage (80) selon la revendication 9, comportant au moins deux capteurs (76, 77) de lumière adaptés à délivrer un signal représentatif du niveau d'éclairement au niveau des deux yeux (1, 2) dudit individu, et dans lequel le niveau moyen de luminance de ladite image ou dudit film et/ou dudit flux lumineux additionnel (F1, F2) est commandé en fonction de ce signal représentatif.

11. Casque de réalité virtuelle (70) destiné à être porté par un individu comportant :
- un dispositif d'affichage (80) selon l'une quelconque des revendications 9 à 10 ;
- des moyens (74, 75, 79) pour maintenir ledit dispositif d'affichage (80) devant les yeux (1, 2) dudit individu ; et
- des moyens (73) pour isoler ledit individu de la lumière ambiante.

## Patentansprüche

1. Verfahren zur Bestimmung eines Lichtfilters, der geeignet ist, auf ein Brillenglas appliziert zu werden, um den Sehkomfort und/oder die Sehleistungen einer die Brille tragenden Person zu verbessern oder aufrechtzuerhalten, das Verfahren umfassend:
a) einen Schritt des Sammelns (11) von Daten bezüglich der Erfordernisse und/oder Gewohnheiten der Person;
b1) einen ersten Schritt des Auswählens (21) mindestens eines Testfilters auf der Grundlage mindestens eines Bewertungskriteriums, wobei das Bewertungskriterium ein Maß der Sehschärfe ist;
(b2) einen zweiten Schritt des Auswählens (22), in Abhängigkeit von den im Schritt (a) gesammelten Daten, mindestens einer Lichtszene, umfassend:
- ein Bild oder einen Film, das bzw. der in Abhängigkeit von den Seherfordernissen und -gewohnheiten der Person bestimmt wird; und
- mindestens eine Lichtquelle, die einen Diskomfort und/oder einen Verlust von Sehleistungen für die nicht mit einem Filter ausgestattete Person erzeugt;
c) einen Schritt des Versetzens der Person in die Situation (31), bei dem der Person die im Schritt b2) ausgewählte Lichtszene visuell durch den im Schritt b1) ausgewählten Testfilter hindurch präsentiert wird;
d) einen Bewertungsschritt (41), der das Messen des Bewertungskriteriums des Schritts b1) beim Versetzen in die Situation des Schritts c) umfasst;
e) einen Schritt des Vergleichens (51) des Ergebnisses der Bewertung des Schritts d) mit einem vorbestimmten Sehschärfeschwellenwert; und
f) einen Schritt des Bestimmens (61) des geeigneten Lichtfilters in Abhängigkeit von dem Ergebnis des Vergleichs des Schritts e).

2. Verfahren zur Bestimmung nach Anspruch 1, bei dem im Schritt b1) der Testfilter in Abhängigkeit von den im Schritt a) gesammelten Daten ausgewählt wird.

3. Verfahren zur Bestimmung nach Anspruch 1 oder 2, bei dem im Schritt f) der Testfilter als der geeignete Lichtfilter berücksichtigt wird, wenn der Vergleich des Schritts e) eine Verbesserung des vordefinierten Kriteriums zeigt.

4. Verfahren zur Bestimmung nach Anspruch 1 oder 2, bei dem im Schritt f), falls der Vergleich des Schritts e) eine Verschlechterung des vordefinierten Kriteriums zeigt, das Verfahren zur Bestimmung im Schritt b1) mit dem Auswählen eines anderen Testfilters wieder aufgenommen wird.

5. Verfahren zur Bestimmung nach einem der Ansprüche 1 bis 4, bei dem im Schritt a) die Person einem Fragebogen unterzogen wird, wobei die gesammelten Daten Antworten auf den Fragebogen umfassen.

6. Verfahren zur Bestimmung nach einem der Ansprüche 1 bis 5, bei dem im Schritt a) auch Messungen bezüglich der Lichtumgebung der Person mittels mindestens eines Lichtsensors erfasst werden.

7. Verfahren zur Bestimmung nach einem der Ansprüche 1 bis 6, bei dem im Schritt b1) der Testfilter nach einem der folgenden Parameter ausgewählt wird:
- ein Niveau der visuellen Transmission;
- ein Transmissionsspektrum in einem gegebenen Wellenlängenband;
- eine räumliche Variation dieser Parameter auf der Oberfläche der Linse; oder
- eine zeitliche Variation dieser Parameter über die Zeit.

8. Verfahren zur Bestimmung nach einem der Ansprüche 1 bis 7, bei dem im Schritt c):
- die im Schritt b2) ausgewählte Lichtszene durch eine Anzeigevorrichtung angezeigt wird; und
- die Person den Testfilter tatsächlich trägt oder aber den Testfilter virtuell trägt, wobei die Lichtszene dann von der Anzeigevorrichtung so angezeigt wird, wie sie von der Person gesehen werden würde, wenn sie den Testfilter tatsächlich tragen würde,
- die Person die Anzeigevorrichtung betrachtet.

9. Anzeigevorrichtung (80) zur Bestimmung eines optimalen Lichtfilters zum Wiederherstellen oder Aufrechterhalten eines Sehkomforts und/oder der Sehleistungen der Person, die Vorrichtung umfassend:
- einen Grafikbildschirm (91), der von einem Mikroprozessor gesteuert wird, um auf dem Grafikbildschirm (91) ein Bild oder einen Film anzuzeigen, das bzw. der von der getesteten Person angesehen werden kann, die einen von dem auf dem Grafikbildschirm (91) angezeigten Bild oder Film kommenden Lichtstrom empfängt,
- ein zusätzliches Beleuchtungssystem (100), das von einem anderen Mikroprozessor oder von demselben Mikroprozessor gesteuert wird, um zu der den Graphikbildschirm (91) betrachtenden Person einen zusätzlichen Lichtstrom (F1, F2) auszusenden, der eine vorbestimmte Energie, spektrale Verteilung, zeitliche Variation, räumliche Verteilung und/oder Winkelverteilung aufweist, wobei das zusätzliche Beleuchtungssystem (100) in Bezug auf den Grafikbildschirm (91) so angeordnet ist, dass das Bild oder der Film ganz oder teilweise sichtbar bleibt,
**dadurch gekennzeichnet, dass** das zusätzliche Beleuchtungssystem (100) dazu geeignet ist, einen zusätzlichen Lichtstrom (F1, F2) auszusenden, der es in Kombination mit dem Grafikbildschirm (80) ermöglicht, eine visuelle Leuchtdichte größer oder gleich 1000 Candela pro Quadratmeter (cd/m²) zu erreichen,
wobei das zusätzliche Beleuchtungssystem (100) Leuchtdioden (101, 102) und einen aktiven oder passiven streuenden Film (110) umfasst, die zwischen den Augen (1, 2) der Person und dem Grafikbildschirm (91) angeordnet sind, wobei der streuende Film (110) geeignet ist, den von den Leuchtdioden (101, 102) emittierten Lichtstrom rückzustreuen.

10. Anzeigevorrichtung (80) nach Anspruch 9, die mindestens zwei Lichtsensoren (76, 77) umfasst, die geeignet sind, ein Signal auszugeben, das für das Beleuchtungsniveau an den beiden Augen (1, 2) der Person repräsentativ ist, und bei der das mittlere Leuchtdichteniveau des Bildes oder des Films und/oder des zusätzlichen Lichtstroms (F1, F2) in Abhängigkeit von diesem repräsentativen Signal gesteuert wird.

11. Virtual-Reality-Headset (70), das dazu bestimmt ist, von einer Person getragen zu werden, umfassend:
- eine Anzeigevorrichtung (80) nach einem der Ansprüche 9 bis 10;
- Mittel (74, 75, 79) zum Halten der Anzeigevorrichtung (80) vor den Augen (1, 2) der Person; und
- Mittel (73) zum Isolieren der Person vom Umgebungslicht.

## Claims

1. Method for determining a light filter suitable for being applied to a spectacle lens in order to improve or maintain the visual comfort and/or the visual performance of an individual who wears said spectacles, said method comprising:
a) a step (11) of collecting data relating to the needs and/or the habits of said individual;
b1) a first selecting step (21) in which at least one trial filter is selected based on at least one evaluation criterion, said evaluation criterion being a measurement of visual acuity;
b2) a second selecting step (22) in which, depending on said data collected in step a), at least one luminous scene is selected, said scene comprising:
- an image or a film determined depending on the visual needs and visual habits of the individual; and
- at least one light source that generates a discomfort and/or a loss of visual performance for the individual not equipped with a filter;
c) a step (31) of exposing said individual to a situation, in which step said luminous scene selected in step b2) is visually presented to said individual through said trial filter selected in step b1);
d) an evaluating step (41) comprising measuring said evaluation criterion of step b1) during the situation exposure of step c);
e) a step (51) of comparing the result of the evaluation of step d) with a predetermined visual-acuity threshold; and
f) a step (61) of determining said suitable light filter depending on the result of the comparison of step e).

2. Determining method according to Claim 1, wherein, in step b1), said trial filter is selected depending on said data collected in step a).

3. Determining method according to Claim 1 or 2, wherein, in step f), said trial filter is adopted as being said suitable light filter if said comparison of step e) demonstrates an improvement in said predefined criterion.

4. Determining method according to Claim 1 or 2, wherein, in step f), if said comparison of step e) demonstrates a degradation in said predefined criterion, the determining method is restarted from step b1) with another trial filter being selected.

5. Determining method according to one of Claims 1 to 4, wherein, in step a), said individual is asked to respond to a questionnaire, the collected data comprising responses to said questionnaire.

6. Determining method according to one of Claims 1 to 5, wherein, in step a), measurements relating to the luminous environment of the individual are also collected by means of at least one light sensor.

7. Determining method according to one of Claims 1 to 6, wherein, in step b1), said trial filter is selected depending on one of the following parameters:
- a visual-transmission level;
- a transmission spectrum in a given wavelength band;
- a spatial variation in these parameters over the area of said lens; or
- a temporal variation in these parameters over time.

8. Determining method according to one of Claims 1 to 7, wherein, in step c):
- said luminous scene selected in step b2) is displayed by a displaying device; and
- said individual actually wears said trial filter or else wears said trial filter virtually, said luminous scene then being displayed by said displaying device such as it would be seen by said individual if he were actually wearing said trial filter,
- said individual observes said displaying device.

9. Displaying device (80) intended to determine an optimal light filter in order to restore or maintain a visual comfort and/or visual performance of the individual, said device comprising:
- a graphical screen (91) that is controlled by a microprocessor in order to display on said graphical screen (91) an image or a film capable of being viewed by said tested individual, who receives a light flux from the image or film displayed on the graphical screen (91),
- an additional illuminating system (100) that is controlled by another microprocessor or by the same microprocessor in order to transmit, to said individual observing said graphical screen (91), an additional light flux (F1, F2) having an energy, a spectral distribution, a temporal variation, a spatial distribution, and/or an angular distribution that is or are predetermined, said additional illuminating system (100) being arranged with respect to said graphical screen (91) so as to let all or some of said image or said film be seen,
**characterized in that** said additional illuminating system (100) is suitable for transmitting an additional light flux (F1, F2) allowing, in combination with said graphical screen (80), a visual luminance higher than or equal to 1000 candelas per square meter (cd/m²), to be achieved,
in which said additional illuminating system (100) comprises light-emitting diodes (101, 102) and an active or passive scattering film (110) placed between the eyes (1, 2) of said individual and said graphical screen (91), said scattering film (110) being suitable for backscattering the light flux emitted by said light-emitting diodes (101, 102).

10. Displaying device (80) according to Claim 9, comprising at least two light sensors (76, 77) that are suitable for delivering a signal representative of the illuminance level at the two eyes (1, 2) of said individual, and wherein the average luminance level of said image or of said film and/or of said additional light flux (F1, F2) is set depending on this representative signal.

11. Virtual reality headset (70) intended to be worn by an individual, comprising:
- a displaying device (80) according to any one of Claims 9 to 10;
- means (74, 75, 79) for keeping said displaying device (80) in front of the eyes (1, 2) of said individual; and
- means (73) for isolating said individual from ambient light.
